(19) Europäisches Patentamt
European Patent Office
Office européen des brevets

(11) **EP 4 613 843 A1**

(12) **EUROPEAN PATENT APPLICATION**
published in accordance with Art. 153(4) EPC

(43) Date of publication:
**10.09.2025 Bulletin 2025/37**

(21) Application number: **23885667.8**

(22) Date of filing: **27.10.2023**

(51) International Patent Classification (IPC):
*C12N 1/02* (2006.01)   *C12M 1/00* (2006.01)

(52) Cooperative Patent Classification (CPC):
**C12M 1/00; C12N 1/02**

(86) International application number:
**PCT/JP2023/038841**

(87) International publication number:
**WO 2024/095911 (10.05.2024 Gazette 2024/19)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority:   **01.11.2022   JP 2022175807**

(71) Applicant: **CANON KABUSHIKI KAISHA
Tokyo 146-8501 (JP)**

(72) Inventors:
• **MATSUDA Takanori
Tokyo 146-8501 (JP)**
• **FURUTA Tatsuo
Tokyo 146-8501 (JP)**
• **FURUI Takaaki
Tokyo 146-8501 (JP)**
• **YOSHIDA Ryoichi
Tokyo 146-8501 (JP)**

(74) Representative: **TBK
Bavariaring 4-6
80336 München (DE)**

(54) **CELL DETACHMENT DEVICE AND CELL DETACHMENT METHOD**

(57)    A cell detachment apparatus that detaches cells placed on a culture surface of a culture container for cells by applying a vibration to the cells, includes a vibrator, and a vibrating plate, wherein the culture container includes a protruding portion on a surface on an opposite side of the culture surface, and wherein the cell detachment apparatus is configured so that in a case where the vibrator, the vibrating plate, an acoustic transmission medium, and the culture container are arranged to be stacked in this order, the acoustic transmission medium and the vibrating plate do not come into contact with the protruding portion.

**FIG.1A**

**FIG.1B**

**Description**

Technical Field

The present invention relates to a cell detachment apparatus and a cell detachment method.

Background Art

In recent years, cell culture for culturing cells for use in regenerative medicine or cells for use in manufacturing a biopharmaceutical is actively performed. The cell culture is broadly classified into an adherent culture and a suspension culture. Particularly, the adherent culture is suitable for the culture of a variety of cells and is widely used. In the adherent culture, to use cultured cells, the step of detaching the cells from a scaffold to which the cells adhere is performed. Accordingly, a detaching condition for collecting cells at a high cell detachment efficiency while reducing damage on cells is considered.

PTL 1 discusses a cell collection apparatus that supplies a detachment solution for decreasing the cell adhesion properties of a cultured cell group to a culture container in which the cultured cell group is stored, and vibrates the culture container using a vibrating device, thereby detaching the cultured cell group from the inner walls of the culture container.

Citation List

Patent Literature

PTL 1: Japanese Patent Application Laid-Open No. 2008-79554

Summary of Invention

Technical Problem

The inventors have given consideration to, through use of a dish including a protruding portion on a bottom surface as a culture container in which a cultured cell group is stored, bringing the dish into contact with a vibrating device. As a result, the inventors have found that, as a vibration transmission path through which a vibration is transmitted from the vibrating device to the cultured cell group stored in the culture container, a plurality of vibration transmission paths exists in the bottom surface and the protruding portion of the dish, and therefore, vibration control is complicated. In a case where a plurality of vibration transmission paths exists, vibrations to be transmitted interfere with each other. Thus, there is a case where the cell detachment efficiency of cells decreases because vibrations to be applied to cells are too strong or too weak.

To address the above issue, the present invention is directed to providing a cell detachment apparatus that detaches cells at a high cell detachment efficiency.

Solution to Problem

A cell detachment apparatus according to the present invention is a cell detachment apparatus that detaches cells placed on a culture surface of a culture container for cells by applying a vibration to the cells. The cell detachment apparatus includes a vibrator, and a vibrating plate, wherein the culture container includes a protruding portion on a surface on an opposite side of the culture surface, and wherein the cell detachment apparatus is configured so that in a case where the vibrator, the vibrating plate, an acoustic transmission medium, and the culture container are arranged to be stacked in this order, the acoustic transmission medium and the vibrating plate do not come into contact with the protruding portion.

A cell detachment apparatus according to the present invention is a cell detachment apparatus that detaches cells placed on a culture surface of a culture container for cells by applying a vibration to the cells. The cell detachment apparatus includes a vibrator, and a vibrating plate, wherein in a case where the vibrator, the vibrating plate, an acoustic transmission medium, and the culture container are arranged to be stacked in this order, the culture container includes a protruding portion on a surface on an opposite side of the culture surface, and wherein a specific gravity of a substance present between the protruding portion and the vibrating plate at a position where the protruding portion and the vibrating plate are closest to each other is greater than 0 and smaller than 0.90.

A cell detachment method according to the present invention is a method for detaching cells placed on a culture surface of a culture container for cells by applying a vibration to the cells. The method includes, through use of a cell detachment apparatus including the culture container including a protruding portion on a surface on an opposite side of the culture surface, an acoustic transmission medium, a vibrator, and a vibrating plate, applying a vibration to the cells in a state where the vibrator, the vibrating plate, the acoustic transmission medium, and the culture container are arranged to be stacked in this order, and applying a vibration to the cells in a state where the acoustic transmission medium and the vibrating plate are not in contact with the protruding portion.

A cell detachment method according to the present invention is a method for detaching cells placed on a culture surface of a culture container for cells by applying a vibration to the cells. The method includes, through use of a cell detachment apparatus including the culture container including a protruding portion on a surface on the opposite side of the culture surface of the culture container, an acoustic transmission medium, a vibrator, and a vibrating plate, applying a vibration to the cells in a state where the vibrator, the vibrating plate, the acoustic transmission medium, and the culture container are arranged to be stacked in this order, and applying a vibration to the cells in a state where a specific gravity of a substance present between the protruding portion and the vibrating plate at a position where the protruding portion and the vibrating plate are closest to each other is greater than 0 and smaller than 0.90.

Advantageous Effects of Invention

According to the present invention, it is possible to provide a vibration detaching apparatus that provides a cell detachment apparatus that detaches cells at a high cell detachment efficiency.

Brief Description of Drawings

[Fig. 1A] Fig. 1A is a top view illustrating an example of a cell detachment apparatus according to an exemplary embodiment of the present invention.
[Fig. 1B] Fig. 1B is a schematic cross-sectional view illustrating an example of the cell detachment apparatus according to the exemplary embodiment of the present invention.
[Fig. 2A] Fig. 2A is a top view illustrating an example of a cell detachment apparatus according to an exemplary embodiment of the present invention.
[Fig. 2B] Fig. 2B is a schematic cross-sectional view illustrating an example of the cell detachment apparatus according to the exemplary embodiment of the present invention2
[Fig. 3A] Fig. 3A is a top view illustrating an example of a cell detachment apparatus according to an exemplary embodiment of the present invention.
[Fig. 3B] Fig. 3B is a schematic cross-sectional view illustrating an example of the cell detachment apparatus according to the exemplary embodiment of the present invention.
[Fig. 4] Fig. 4 is a schematic diagram of a culture container according to the exemplary embodiment of the present invention.
[Fig. 5] Fig. 5 is a schematic diagram of silicone rubber having a central convex shape according to the exemplary embodiment of the present invention.
[Fig. 6] Fig. 6 is a schematic diagram of a vibrator according to the exemplary embodiment of the present invention.
[Fig. 7A] Fig. 7A is a top view of a positioning jig according to the exemplary embodiment of the present invention.
[Fig. 7B] Fig. 7B is a side view of the positioning jig according to the exemplary embodiment of the present invention.

Description of Embodiments

Cell detachment apparatuses according to exemplary embodiments of the present invention will be described below with examples. The exemplary embodiments of the present invention, however, are not limited to the following examples.

(Basic Configuration)

A cell detachment apparatus 10 according to an exemplary embodiment of the present invention includes a vibrator 12 and a vibrating plate 11. In a case where the cell detachment apparatus 10 applies a vibration to cells, the vibrator 12, the vibrating plate 11, an acoustic transmission medium 13, and a culture container 21 are arranged to be stacked in this order. The cell detachment apparatus according to the exemplary embodiment of the present invention detaches cells placed on a culture surface of the culture container 21 for cells by applying a vibration to the cells.

A configuration may be employed in which the cell detachment apparatus 10 includes other members between the vibrator 12, the vibrating plate 11, the acoustic transmission medium 13, and the culture container 21. The vibration to be applied to the cells may be a vibration in the ultrasonic range. The vibration in the ultrasonic range is a vibration at a frequency of 20 kHz or more. A configuration may be employed in which the acoustic transmission medium 13 is installed in advance in the cell detachment apparatus 10. A configuration may be employed in which the cell detachment apparatus 10 does not include the acoustic transmission medium 13, and a user installs the acoustic transmission medium 13 in the cell detachment apparatus 10. Alternatively, the cell detachment apparatus 10 may include a control unit that drives the vibrator 12 by applying a voltage to the vibrator 12, thereby applying a vibration to cells. The acoustic transmission medium can also be referred to as an "acoustic transmission material", an "acoustic matching material", and a "transmission medium".

"Detaching the cell" refers to weakening the adhesion of the cell to the cell culture container and achieving the state where the cell can be collected. At this time, the detached cell may be in the state where the cell is disengaged as an individual cell, or may be in the state where a plurality of cells adheres to each other. Alternatively, as will be described below, the detached cell may be in the form of a sheet.

The cell detachment apparatus includes the vibrator and the vibrating plate as a vibration generation unit. A vibration mechanism generates a bending vibration obtained by amplifying a vibration according to the combination of the hardness of the vibrator including a piezoelectric material that expands and contracts itself and the hardness of the vibrating plate bonded to the vibrator. The vibration mechanism uses resonance caused by the structure of the vibrator and can obtain a large vibration at a resonance frequency.

(Culture Container)

Examples of a container for the purpose of cell culture widely used in the cell culture field include a dish, a flask, and a well plate. Particularly, as the dish, dishes manufactured by Corning Incorporated, Thermo Fisher Scientific Inc., and AGC Techno Glass Co., Ltd. (Iwaki) in Japan are used as general-purpose disposable containers.

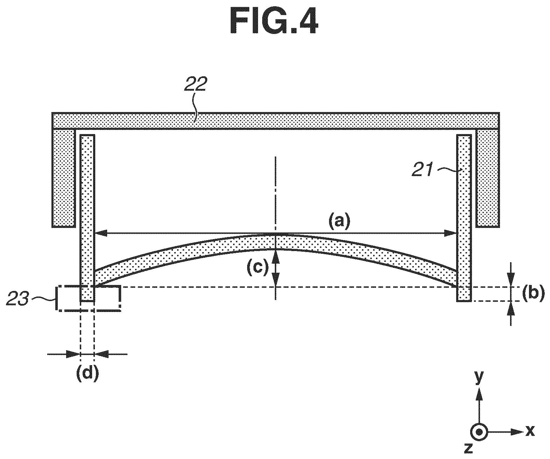

The shape of the dish of each company as the culture container in which cells are cultured is as illustrated in a schematic diagram in Fig. 4. The dimensions of the dish are as illustrated in table 1. A culture surface refers to a surface surrounded by the side walls of the dish. In a case where cells are cultured, the cells adhere to the culture surface. In table 1, (a) indicates the inner diameter of the dish, (b) indicates the length of a protruding portion, (c) indicates the warpage height of a bottom surface, and (d) indicates the width of the protruding portion. The inner diameter of the dish is the diameter of a region inside a protruding portion 23 located in an end portion of the bottom surface of the dish in the bottom surface of the dish. The protruding portion 23 is provided on the bottom surface of the dish that is a surface on the opposite side of the culture surface of the culture container, and protrudes in a direction approximately perpendicular to the bottom surface of the dish. The protruding portion 23 may be provided in a circular shape in the end portion of the bottom surface of the dish, or may include a cutout.

Possible examples of the main function of the protruding portion 23 include preventing the bottom surface of the culture container from being scratched, enabling containers to be arranged to be stacked, and preventing the temperature of a worktable from being directly transmitted to the bottom surface of the culture container when the culture container is placed on the worktable. The warpage height of the bottom surface indicated by (c) not only greatly varies among lots, but also greatly varies among culture containers in each lot. The dimensions of the culture container 21 are not limited to the dimensions illustrated in table 1, and the culture container 21 of any size can be used. In a case where the protruding portion 23 of the culture container 21 comes into contact with the vibrating plate 11 during driving, a vibration waveform formed in the vibrating plate 11 is reduced, and a vibration amplitude required for cell detachment is not obtained. This causes a decrease in the cell detachment efficiency, which is not desirable.

As the container for the purpose of cell culture, a dish, a flask, or a well plate as a general-purpose product is widely used. In the present invention, however, the container for the purpose of cell culture is not particularly limited. The material of the culture container may be a material that is chemically stable and enables desired cells to be cultured at that time. Examples of the material of the culture container include at least one selected from the group consisting of polyethylene, polypropylene, polycarbonate, polystyrene, polyvinyl chloride, nylon, polyurethane, polyurea, polylactic acid, polyglycolic acid, polyvinyl alcohol, polyvinyl acetate, poly(meth)acrylic acid, a poly(meth)acrylic acid derivative, polyacrylonitrile, poly(meth)acrylamide, a poly(meth)acrylamide derivative, a polysulfone, cellulose, a cellulose derivative, polysilicone, polymethylpentene, glass, and a metal. Among these, polystyrene is desirable.

[Table 1]

| Name | Manufacturer | (a) Inner Diameter | (b) Length of Protruding Portion | (c) Warpage Height of Bottom Surface | (d) Width of Protruding Portion |
|---|---|---|---|---|---|
| | | mm | mm | mm | mm |
| 60-mm Dish | Corning Incorporated | 51.6 | 0.5 | 0.150 - 0.27 | 0.66 |
| 60-mm Dish Iwaki | AGC Techno Glass Co., Ltd. | 52.1 | 0.5 | 0.100 - 0.17 | 0.7 |

(continued)

| Name | Manufacturer | (a) Inner Diameter | (b) Length of Protruding Portion | (c) Warpage Height of Bottom Surface | (d) Width of Protruding Portion |
|---|---|---|---|---|---|
| | | mm | mm | mm | mm |
| 60-mm Dish Nunc | Thermo Fisher Scientific Inc. | 52.2 | 0.5 | -0.005 - 0.005 | 0.7 |

(First Exemplary Embodiment)

(Configuration of Apparatus)

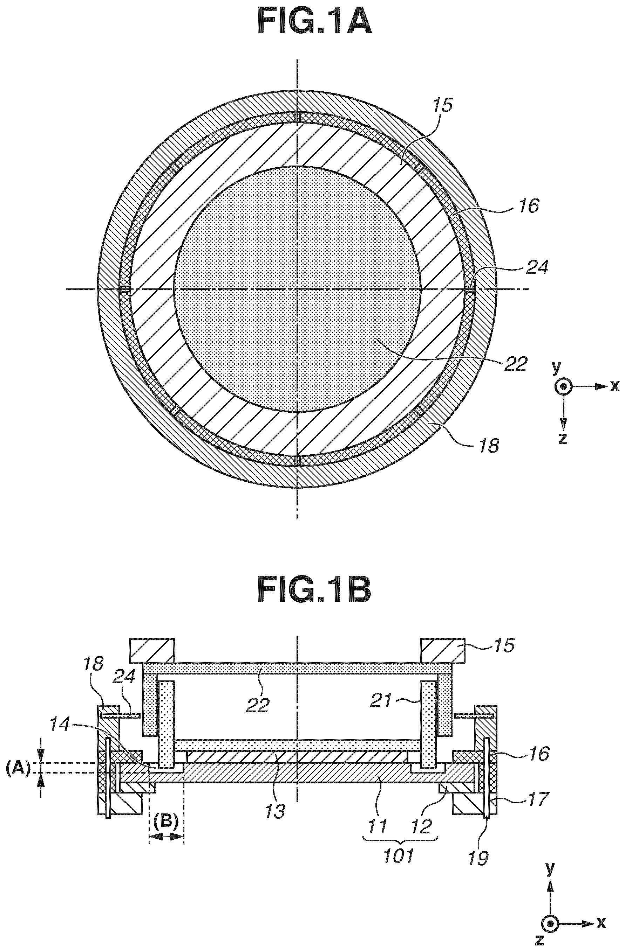

Figs. 1A and 1B illustrate a cell detachment apparatus according to a first exemplary embodiment of the present invention.

The cell detachment apparatus includes a vibrator 12 and a vibrating plate 11 as a vibration generation unit 101. A vibration mechanism generates a bending vibration obtained by amplifying a vibration according to the combination of the hardness of the vibrator 12 including a piezoelectric material that expands and contracts itself and the hardness of the vibrating plate 11 bonded to the vibrator 12. The vibration mechanism uses resonance caused by the structure of the vibrator and can obtain a large vibration at a resonance frequency.

Although Figs. 1A and 1B illustrate a ring type vibrator as a typical vibration generation unit 101, a Langevin type transducer or a rectangular transducer, which can obtain a large amplitude, may be used.

To efficiently transmit a vibration formed by resonance in the vibrating plate 11 to a culture container 21 in which cells are cultured, an acoustic transmission medium 13 is interposed between the vibrating plate 11 and the culture container 21. If the culture container 21 is installed directly on the vibrating plate 11, contact portions between the culture container 21 and the vibrating plate 11 are in point contact, and the vibration is consumed as energy other than the vibration, such as an abnormal noise or the occurrence of heat generation. This causes the deterioration of efficiency.

It is desirable that the acoustic transmission medium 13 basically be installed in a sufficiently wide area on a bottom surface of the culture container 21. Specifically, it is desirable that a close contact area be greater than or equal to 30% and less than 100%. The close contact area refers to the proportion of the area of a region where the acoustic transmission medium 13 and the culture container 21 are in contact with each other to the area of a region inside a protruding portion 23 of the bottom surface of the culture container 21. If the close contact area is close to 100%, a sufficiently high cell detachment efficiency is obtained based on the effect of weakening the binding force between cells on the culture container 21 having received an inertial force due to a vibration generated in the vibrating plate 13 in a standing wave mode as a vibration mode.

However, the shape of the bottom surface of the container of each company as a general-purpose product as the culture container 21 varies, and there is a case where the acoustic transmission medium 13 cannot be completely brought into close contact with the culture container 21 even by causing the acoustic transmission medium 13 to follow the shape of the culture container 21. If the close contact area is at least 30%, the combination of a traveling wave mode as a vibration mode with the standing wave mode is effective. By shear stress due to a flowage having a large flow in the radial direction that occurs in a detachment solution in the traveling wave mode, it is possible to detach cells in a region with which the acoustic transmission medium 13 is not in direct contact.

To fix the vibrating plate 11 and the vibrator 12, a structure is employed where an upper cover 18, a lower cover 17, a buffer material 16, and a bolt 19 in Figs. 1A and 1B sandwich end portions of the vibrating plate 11 and the vibrator 12. However, pressure to sandwich the end portions tightens the end portions without inhibiting the vibration generated in the vibrating plate 11. As a buffer material (not illustrated), a member including felt or sponge may be provided between the vibrator 12 and the lower cover 17 or on the lower side of the lower cover 17. The provision of the buffer material (not illustrated) can make the vibration of the vibrating plate 11 and the vibrator 12 less likely to be reduced due to contact with another member.

If an air layer enters between the culture container 21 and the acoustic transmission medium 13, the air layer reflects ultrasound, and therefore, the vibration is not efficiently transmitted to the culture container 21. Thus, to increase the close contact between the culture container 21 and the acoustic transmission medium 13, a weight 15 having a ring shape is installed on a culture container upper lid 22. In the case of a 60-mm dish, it is desirable that the weight of the weight 15 be from 100 g to 600 g. If the weight 15 is too heavy, the weight 15 holds down and inhibits the vibration itself. Additionally, the culture container 21 itself also sags, whereby the vibration transmission state becomes non-uniform. This causes a decrease in the cell detachment efficiency. At this time, a load using the weight 15 is desirable in consideration of the insulation properties of the vibration. However, a loading method using a spring or a leaf spring may be used insomuch that the vibration is not inhibited.

Using an alternating-current power supply that supplies a voltage having a resonance frequency, a voltage which has a frequency in a vibration mode where a desired vibration waveform can be formed on a cell culture surface of the culture container 21 and with which a desired amplitude can be obtained is applied to the vibrator 12, thereby driving the cell detachment apparatus. At this time, if a voltage in a phase S that is a sensor phase included in the vibrator 12 is measured using an oscilloscope, it is possible to directly monitor a voltage generated by the vibrator 12 itself distorting. Thus, it is possible to drive the cell detachment apparatus while confirming the vibration state of the vibration generation unit 101. A thermocouple capable of measuring the temperature during a cell detachment step is also installed in a portion that does not influence the vibration, whereby it is possible to drive the cell detachment apparatus while monitoring the temperature. An infrared temperature measuring device may be used to measure temperature.

(Vibrating Plate)

It is desirable that the vibrating plate 11 included in the vibration generation unit 101 according to the exemplary embodiment of the present invention include at least one selected from the group consisting of glass, metal, and quartz. The metal used for the vibrating plate 11 may be stainless steel (SUS) or aluminum. The vibrating plate 11 is made of glass, a metal, or quartz, whereby it is possible to output a large amplitude at a relatively high driving frequency in the ultrasonic region without breaking the vibrating plate 11.

In the case of the vibration generation unit 101 including the vibrating plate 11 having a disk shape and the vibrator 12 having a ring shape, it is desirable that the outer diameter dimension of the vibrating plate 11 be equal to the outer diameter dimension of the vibrator 12. It is desirable that, in a case where the vibrator 12 and the vibrating plate 11 are bonded together and generate a bending vibration, the midpoint in the thickness direction of the bending, i.e., a neutral surface that neither stretches nor compresses in the bending, be on the vibrating plate side to efficiently use the distortion of the vibrator 12 for the bending.

The vibrating plate 11 may also include a marker (a label, a print, or an engraved mark) indicating the position where the acoustic transmission medium 13 is to be placed on the vibrating plate 11. The vibrating plate 11 includes the marker, whereby the user can easily place the acoustic transmission medium 13 at an appropriate position.

In the first exemplary embodiment, a structure is employed where the protruding portion 23 of the culture container 21 does not come into contact with the vibrating plate 11 and the acoustic transmission medium 13. As illustrated in the schematic diagrams in Figs. 1A and 1B, a groove 14 is provided in a region including the position where the position of the protruding portion 23 is projected onto the vibrating plate 11 in the direction in which the vibrator 12, the vibrating plate 11, the acoustic transmission medium 13, and the culture container 21 are arranged to be stacked in this order (a y-direction in Figs. 1A and 1B). Consequently, the vibration is not directly transmitted from the vibrating plate 11 to the culture container 21, and the vibration can be transmitted only from the acoustic transmission medium 13 to the bottom surface of the culture container 21.

That the protruding portion 23 of the culture container 21 and the vibrating plate 11 do not come into contact with each other means that the protruding portion 23 of the culture container 21 does not substantially come into direct contact with a vibrating surface of the vibrating plate 11. It is desirable that the protruding portion 23 be in midair. A buffer material capable of preventing the vibration of the vibrating plate 11 from being transmitted to the culture container 21 through the protruding portion 23 may be present. That is, the cell detachment apparatus may be configured to bring at least one of the acoustic transmission medium and the vibrating plate into contact with the protruding portion through the buffer material, and prevent the acoustic transmission medium and the vibrating plate from coming into contact with the protruding portion not through the buffer material. It is desirable that the buffer material be at least one of felt and sponge.

Further, in other words, it is desirable that the specific gravity of a substance present between the protruding portion and the vibrating plate at the position where the protruding portion and the vibrating plate are closest to each other be greater than 0 and smaller than 0.90. The specific gravity of the substance present between the protruding portion and the vibrating plate is smaller than 0.90, whereby the vibration is less likely to be transmitted. Thus, it is possible to reduce the interference of the vibration. It is desirable that the specific gravity of the substance present between the protruding portion and the vibrating plate be smaller than 0.80. It is desirable that the specific gravity of the substance present between the protruding portion and the vibrating plate be smaller than 0.50.

For example, the specific gravity is calculated by a pycnometer method. In an environment where temperature and pressure are constant, a mass W0 of a pycnometer that is empty, a mass W1 of the pycnometer filled with water, and a mass W of the pycnometer filled with a sample are measured. A specific gravity S of the sample is calculated by the following formula.

$$S = (W - W0)/(W1 - W0) \quad \text{(formula 1)}$$

It is desirable that the depth of the groove 14 indicated by (A) in Fig. 1B be greater than the length of the protruding portion 23 ((b) in Fig. 4). It is desirable that the width of the groove 14 indicated by (B) in Fig. 1B be greater than the width of the protruding portion 23 ((d) in Fig. 4).

The depth of the groove 14 refers to the depth of the groove 14 in the direction in which the vibrator, the vibrating plate, the acoustic transmission medium, and the culture container are arranged to be stacked. The length of the protruding portion 23 refers to the length of the protruding portion 23 in the direction in which the vibrator, the vibrating plate, the acoustic transmission medium, and the culture container are arranged to be stacked. The width of the groove 14 refers to the width of the groove 14 in a direction perpendicular to the direction in which the vibrator, the vibrating plate, the acoustic transmission medium, and the culture container are arranged to be stacked. The width of the protruding portion 23 refers to the width of the protruding portion 23 in the direction perpendicular to the direction in which the vibrator, the vibrating plate, the acoustic transmission medium, and the culture container are arranged to be stacked.

Specifically, as illustrated in table 1 illustrating a list of the dimensions of the culture containers, since the length of the protruding portion 23 of each culture container 21 is from 0.5 mm to 0.6 mm, the depth of the groove 14 needs to be greater than 0.6 mm. Although the width of the groove 14 needs to be wider than 0.7 mm, which is the width of the protruding portion 23, it is desirable that the width of the groove 14 be greater than or equal to 3.0 mm in consideration of the positional accuracy of the installation of the culture container 21. The excessive depth and width of the groove 14 may cause a decrease in the strength of the vibrating plate 11 or a decrease in the amplitude of a desired vibration mode. Thus, it is desirable that the depth of the groove 14 be shallower than 1.5 mm, and the width of the groove 14 be narrower than 7.0 mm. In an upper boundary portion between the vibrating surface and the groove 14, a taper may be formed. The formation of the taper provides the effect of preventing the groove 14 from interfering with the culture container 21 when the culture container 21 is tilted, or the effect of relieving a stress concentration that occurs in the vibrating plate 11.

(Acoustic Transmission Medium)

As a requirement for the acoustic transmission medium 13, it is necessary to match the acoustic transmission medium 13 to the vibrating plate 11 that generates a vibration and the culture container 21 as a target to which the vibration is transmitted. For the matching, the physical property matching of acoustic impedance and also the mechanical matching of close contact are necessary. Particularly, since the cell detachment apparatus according to the exemplary embodiment of the present invention forms the vibration waveform of the vibrating plate 11 on the cell culture surface of the culture container 21, the mechanical matching is important so that the acoustic transmission medium 13 can come into close contact with the culture container 21 in a wide area.

If a commercial dish-shaped container is used as the culture container 21, a low-molecular material such as glycerin or water, which easily deforms by a load, can be used as the acoustic transmission medium 13. At this time, when the culture container 21 is placed on the vibrating plate 11, there is a case where the protruding portion 23 of the culture container 21 comes into direct contact with the vibrating plate 11, a plurality of vibration transmission paths exists, generated vibrations become complicated, and it is difficult to control the vibrations.

In the cell detachment apparatus according to the exemplary embodiment of the present invention, to independently control a vibration, the protruding portion 23 of the culture container 21 does not come into direct contact with the vibrating plate 11. Further, to efficiently transmit the vibration of the vibrating plate 11 to the bottom surface of the culture container 21, a polymeric material capable of supporting the culture container 21, such as rubber or gel, is used as the acoustic transmission medium 13. Specifically, the polymeric material that can be used is a material including at least one selected from the group consisting of silicone resin, urethane resin, diallyphthalate resin, unsaturated polyester, epoxy resin, phenolic resin, urea resin, melamine resin, and natural rubber. It is desirable that the acoustic transmission medium 13 include silicone rubber, because the silicone rubber has a physical property that attenuates little in the frequency range of driving frequencies, and therefore has a high transmission efficiency, and further, the silicone rubber can reduce the heat generation of the acoustic transmission medium itself.

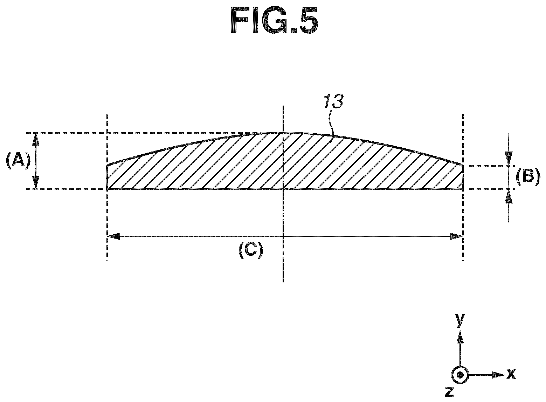

From table 1, it is understood that the shape of the bottom surface of the container as each culture container 21 is not simply a flat surface, but is warped. Based on this, to bring the acoustic transmission medium 13 into close contact with the culture container 21 in a wide area, the acoustic transmission medium 13 needs to follow the shape of the bottom surface. That is, it is desirable that the surface of the acoustic transmission medium 13 that comes into contact with the culture container 21 includes a convex curved surface. For example, in the case of a 60-mm diameter dish manufactured by AGC Techno Glass Co., Ltd. in table 1, the warpage of the bottom surface is 0.10 mm to 0.70 mm. In this case, for example, silicone rubber that is a polymeric material with a hardness of 10° having a central convex shape and having a center height (A) of 0.8 mm, a side surface height (B) of 0.4 mm, and a diameter (C) of 48 mm as illustrated in Fig. 5 is used as the acoustic transmission medium 13. At this time, the close contact area is 60%, and when the weight 15 of 300 g is placed, the close contact area is 90% due to the compression of the polymeric material as the acoustic transmission medium 13 and the sagging of the culture container 21.

To obtain a wide close contact area, it is desirable that the center of the polymeric material of the acoustic transmission medium 13 and the center of the culture container 21 overlap with each other. As an example of the method for aligning the acoustic transmission medium 13 and the culture container 21, a positioning jig will be described below. However, not only the positioning jig but also another method may be used.

It is desirable that the outer surface of the surface of the acoustic transmission medium 13 that comes into contact with the culture container 21 has recesses and projections. The outer surface has recesses and projections, whereby, in a case where the weight 15 is placed, the projection portions are compressed, and as a result, the acoustic transmission medium 13 can come into close contact with the culture container 21 in a wide area. Further, this also has the effect that after high cell detachment performance is exerted, if the weight 15 is removed, the acoustic transmission medium 13 and the culture container 21 can be easily detached.

Similarly, for the purpose of facilitating the detachment of the acoustic transmission medium 13 and the culture container 21, the surface of the polymeric material of the acoustic transmission medium 13 that comes into contact with the culture container may be coated with fluorine.

To directly observe the cell detachment situation, it is desirable that the acoustic transmission medium 13 be transparent.

(Vibrator)

Although in the first exemplary embodiment, the vibrator 12 having a ring shape is used, the shape of the vibrator 12 that bends the vibrating plate 11 may be a ring shape or a disk shape. To directly observe the cell detachment situation, it is desirable that the shape of the vibrator 12 be a ring shape capable of securing the field of view of a lower portion of the culture container 11.

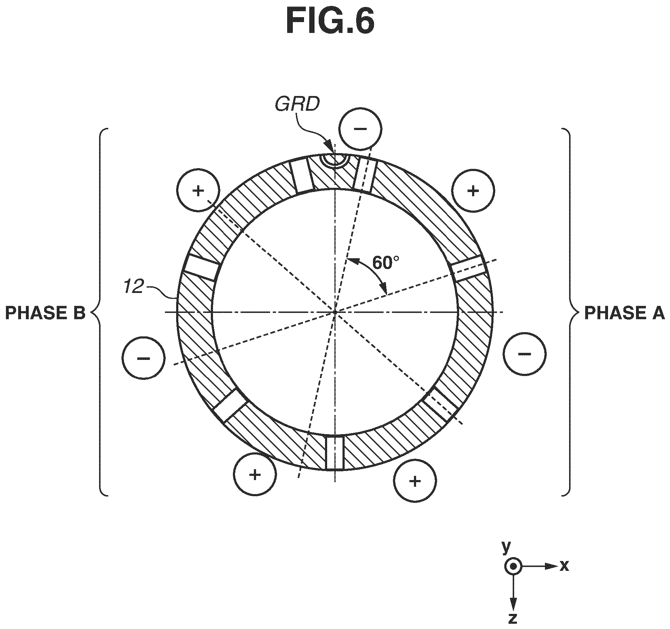

The vibrator 12 exerts piezoelectric properties by being subjected to a polarization process. As illustrated in Fig. 6, however, the polarity of the polarization may be changed according to the electrode pattern. The vibrator 12 subjected to the polarization process can excite the standing wave mode or the traveling wave mode as a vibration mode by controlling the phase of an input alternating-current voltage with respect to each electrode pattern. An alternating-current voltage with a phase difference of 180 degrees with respect to a positively polarized electrode is applied to a negatively polarized electrode, whereby a standing wave is generated. In contrast, as illustrated in Fig. 6, in a case where electrodes are divided into a phase A and a phase B to left and right, and if an alternating-current voltage with a phase difference of 90 degrees between the phases A and B is applied, a traveling wave for two-phase driving is generated.

Although the electrode pattern is formed of silver (Ag) by a printing method, the electrode material may be a noble metal such as gold (Au), platinum (Pt), or palladium (Pd) or a base metal such as copper (Cu) besides Ag. The formation method may be a printing method, a plating method, or a sputtering method.

As the composition of the piezoelectric material included in the vibrator 12 used for the vibration generation unit 101, lead zirconate titanate ($PbZrTiO_3$) (PZT) is used. It is, however, desirable that the piezoelectric material be a lead-free piezoelectric material that does not substantially use lead (Pb), in consideration of environmental regulations. Examples of the lead-free piezoelectric material include at least one selected from the group consisting of materials having barium titanate ($BaTiO_3$) (BT), sodium niobate ($NaNbO_3$), bismuth sodium titanate ($BiNaTiO_3$), and bismuth ferrite ($BiFeO_3$) as main components. A metal element may be added to each of the combinations of these or each of the main components. Particularly, vibration performance comparable with that of $PbZrTiO_3$ (PZT) is confirmed in a $BaTiO_3$ (BT) material. As a material other than a ceramic, a monocrystalline material or a polymer piezoelectric material may be used.

It is desirable that, in a case where the vibrator 12 and the vibrating plate 11 that are bonded together generate a bending vibration, the midpoint in the thickness direction of the bending, i.e., a neutral surface that neither stretches nor compresses in the bending, be on the vibrating plate 11 side to efficiently use the distortion of the vibrator 12 for the bending. Thus, the combination of the thickness of the vibrator 12 and the thickness of the vibrating plate 11 is set based on the relationship between the hardness of the vibrator 12 and the hardness of the vibrating plate 11.

(Positioning Jig)

When the culture container 21 is installed in the cell detachment apparatus, the center of the culture container 21 and the centers of the vibrating plate 11 and the acoustic transmission medium 13 of the cell detachment apparatus are caused to coincide with each other, whereby, even if the bottom surface of the culture container 21 is warped, it is possible to form a close contact state where an air layer is not present between the culture container 21 and the acoustic transmission medium 13.

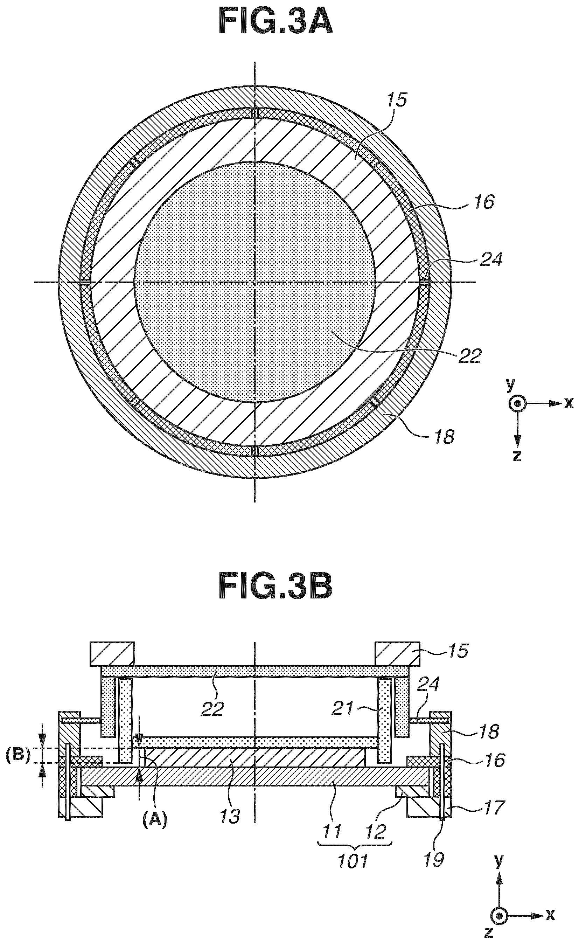

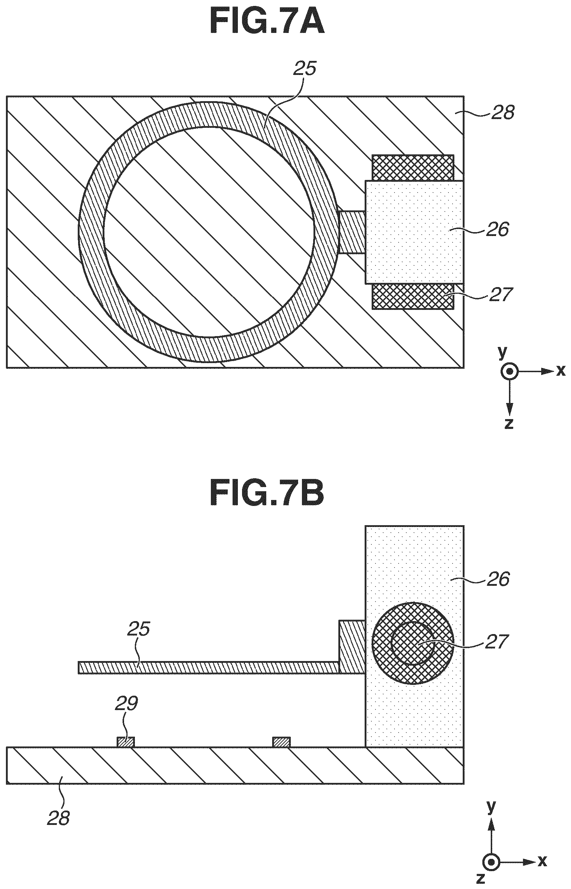

As illustrated in Figs. 1A, 1B, 3A, and 3B, an outer peripheral portion of the culture container 21 is hit against a positioning pin 24, whereby it is possible to align the center of the culture container 21. As illustrated in Figs. 7A and 7B, the culture container 21 may be installed in an annular holding ring 25 of which the center position is determined. The culture container 21 is moved up and down using a dial 27 of a Z-axis stage bracket 26, whereby it is possible to accurately install the culture container 21 relative to the center of the acoustic transmission medium 31 positioned using a pedestal positioner 29 of a bottom plate 28 of a positioning jig 102.

If the centers of the acoustic transmission medium and the vibrating plate 11 do not accurately coincide with each other without using the positioning jig, there is a possibility that the protruding portion 23 of the culture container 21 comes into contact with the vibrating plate 11 during driving. At this time, a vibration waveform formed in the vibrating plate 11 is reduced, and a vibration amplitude required for cell detachment is not obtained. This causes a decrease in the cell detachment efficiency.

(Driving of Apparatus)

A vibration waveform that can be formed by the cell detachment apparatus has the standing wave mode where a vibration waveform is formed in concentric circles from the center of the vibrating plate 11 having a disk-like shape, and the traveling wave mode where a vibration waveform in the radial direction is formed on the vibrating plate 11 by vibrating the vibrator 12 while changing phases in the radial direction. In each mode, a vibration waveform can be formed by applying a voltage at a resonance frequency to the vibrator 12. The resonance frequency and the vibration waveform shape of the vibration waveform are calculated by structural calculation using the shape of the vibrator 12 and the material physical property values such the hardness and the density of the vibrator 12.

As a vibration waveform that can be used for cell detachment, any vibration waveform with which the cell culture surface of the culture container 21 can be vibrated at a constant amplitude, e.g., an amplitude of 1 $\mu$m, can be used. Basically, when a frequency sweep is performed from a low-frequency wave to a high-frequency wave, e.g., from 20 kHz to 150 kHz in the ultrasonic region, vibrations that resonate can be detected. The amplitude of a vibration detected on the low-frequency wave side is large, but the acceleration of the vibration is small. In contrast, the amplitude of a vibration detected on the high-frequency wave side is small, but the acceleration of the vibration is large.

A condition for applying a force to weaken the adhesive force of a cultured cell to the culture container 21 greatly depends on the adhesive force of the cell and the size and the shape of the cell. Further, a flowage that occurs in a detachment solution also differs depending on the amplitude and the acceleration, and therefore, a variety of combinations of flowages exist. Simply, a large amplitude and a large acceleration promote the occurrence of cavitation that causes a decrease in the survival rate of cells, which is not desirable.

If the vibrator 12 is continuously driven, the resonance frequency shifts to the low-frequency wave side due to the heat generation of the vibrator 12 itself. Thus, if the vibrator 12 continues to be driven by fixing the frequency, the amplitude becomes small. Thus, the vibrator 12 is driven using a driving method for providing a suspension time after vibrating the vibrator 12 for a certain time, and reducing a temperature rise due to self-heating, whereby it is possible to obtain a sufficient amplitude. Alternatively, the vibrator 12 is driven using a driving method for repeating a sweep from the high-frequency wave side to the low-frequency wave side with a certain frequency width and always passing through a resonance frequency even if the vibrator 12 generates heat, whereby it is possible to obtain a sufficient amplitude.

(Evaluation of Vibration of Vibrator)

The vibration waveform of the vibrating plate of the cell detachment apparatus can be measured using a laser Doppler vibrometer (AT7200 manufactured by Graphtec Corporation). To measure the vibration waveform, the cell detachment apparatus is driven at a desired resonance frequency and a desired voltage, and the outer surface of the vibrating plate 11 is two-dimensionally scanned in an XY direction using a laser. At this time, for example, the outer surface of the vibrating plate 11 is scanned at 1-mm intervals. The vibration velocity at each point is obtained by the scanning, and the amplitude in a Z-direction can be calculated by calculation. As a result, it is possible to three-dimensionally configure the vibration waveform of the vibrating plate 11. Thus, it is possible to dynamically grasp the positions of an antinode where the amplitude of the vibration in driving is large, and a node where the vibrating plate 11 does not vibrate. In the outer surface of the vibrating plate, the position where the amplitude of the vibration is a local maximum is an antinode portion, and the position where the amplitude of the vibration is local is a node portion.

The vibration of the cell culture surface of the culture container 21 can also be measured with a similar configuration. At this time, the acoustic transmission medium 13 is installed on the vibrating plate 11. A film of a metal such as Ag is formed on the inner surface of the culture container 21 by a vapor deposition method so that a laser is reflected by the culture container 21. The acoustic transmission medium 13 needs to be installed to prevent an air layer from entering between the culture container 21 and the acoustic transmission medium 13. At this time, as the method for preventing the entry of an air layer, the culture container 21 and the acoustic transmission medium 13 may be vacuumed in a vacuum chamber.

In a case where resonance for the purpose of driving the cell detachment apparatus is in the standing wave mode where the culture container 21 vibrates in concentric circles, a sweep is performed near a resonance frequency for this purpose, and the amplitude of a central portion of the culture container 21 is measured. Further, the entirety of the culture surface of the culture container 21 is two-dimensionally scanned at a frequency having the largest amplitude, whereby it is possible to three-dimensionally grasp a vibration waveform that occurs on the culture surface of the culture container 21.

(Culture Conditions for Cells)

Culture conditions for cells can be appropriately selected according to cells to be cultured. Generally, an appropriate culture medium is added into the culture container 21, and cells are seeded at about $1.0 \times 10^1$ to $5.0 \times 10^4$ cells/cm$^2$ in the culture medium and cultured under an environment with a temperature of 37°C and a $CO_2$ concentration of 5%. At this time, it is desirable to culture cells until the cell occupancy area ratio of the culture container 21 is about seven to eight tenths, i.e., the cells are in a so-called subconfluent state. Cells may be cultured until the cells enter a confluent state where the cells cover the culture surface, and the cells may be in the state where the cells are cultured in the form of a sheet (a cell sheet).

(Detachment Test)

The cell detachment apparatus can be applied to a variety of types of cells. Examples of the cells include at least one selected from the group consisting of a Chinese hamster ovary (CHO)-derived cell, a mouse connective tissue L929 cell, a mouse skeletal muscle myoblast cell (a C2C12 cell), a human embryonic lung-derived normal diploid fibroblast cell (a TIG-3 cell), a human embryonic kidney-derived cell (a HEK293 cell), a human alveolar basement epidermal gland cancer-derived A549 cell, a human cervical cancer-derived HeLa cell, an epithelial cell, an endothelial cell, a skeletal muscle cell, a smooth muscle cell, a cardiac muscle cell, a neuronal cell, a glial cell, a fibroblast cell, a liver parenchymal cell, a liver non-parenchymal cell, a fat cell, an induced pluripotent stem (iPS) cell, an embryonic stem (ES) cell, an embryonic germ (EG) cell, an embryonic cancer (EC) cell, a mesenchymal stem cell, a liver stem cell, a pancreatic stem cell, a skin stem cell, a muscle stem cell, a germ stem cell, progenitor cells for tissues, and cells differentiation-induced from these.

In a cell detachment step, it is necessary to replace a culture solution used in the step of culturing cells with a detachment solution. Normally, the detachment solution contains a proteolytic enzyme. Examples of the proteolytic enzyme include at least one selected from the group consisting of trypsin, Accutase, collagenase, natural protease, chymotrypsin, elastase, papain, pronase, and recombinants of these. However, there is concern about the possibility that the proteolytic enzyme breaks down membrane proteins included in the cells. There is a possibility that the breakdown of the membrane proteins decreases properties such as the adhesion of the cells. **In** contrast, a cell detachment technique according to the present invention does not use a proteolytic enzyme, or even if the cell detachment technique uses a proteolytic enzyme, exerts a similar effect with a very small amount of the proteolytic enzyme. Thus, the cell detachment technique has the effect of reducing the breakdown of the membrane proteins in the cells. To make the membrane proteins in the cells less likely to decrease, the detachment solution may include a culture medium component.

The culture container 21 in which the culture solution is replaced with the detachment solution is installed in the cell detachment apparatus, and the cell detachment apparatus is driven in the state where the cell detachment apparatus is held at a constant temperature, e.g., 37°C, in an incubator. The cell detachment apparatus is driven for a driving time having a sufficient length for cell detachment. After the cell detachment apparatus is driven, detached cells are collected using a dropper, and cells adhering to the culture container 21 are collected by a pipetting method. The cell detachment efficiency is the proportion of the number of cells detached by the vibration of the cell detachment apparatus to the total number of the number of cells detached by the vibration of the cell detachment apparatus and the number of cells detached by the pipetting method. The survival rate is the proportion of surviving cells among the cells collected by the vibration of the cell detachment apparatus. Residual cells remaining on the bottom surface of the culture container 21 after cells are detached can be observed using scattered light from a backlight.

(Example 1)

Example 1 of the first exemplary embodiment is illustrated below.

In example 1, the cell detachment apparatus in Figs. 1A and 1B was used. As the culture container 21, a 60-mm diameter dish manufactured by Corning Incorporated in table 1 was used. As the vibrating plate 11 of the cell detachment apparatus, glass was used which had an outer diameter of 70 mm and a thickness of 4 mm and in which the groove 14 was formed so that the protruding portion 23 of the culture container 21 did not come into contact with the vibrating plate 11. The dimensions of the groove 14 were an inner diameter of 50 mm, a width of 5 mm, and a depth of 1 mm. The material composition of the piezoelectric material included in the vibrator 12 was PZT. The vibrator 12 had an outer diameter of 70 mm, which was the same as that of the vibrating plate, and an inner diameter of 57 mm. The vibrator 12 had a thickness of 2 mm, considering that the neutral surface was on the vibrating plate 11 side. On the vibrating plate 11, as the acoustic transmission medium 13, silicone rubber with a hardness of 30° having a central convex disk shape and having a center height (A) of 0.9 mm, a side surface height (B) of 0.4 mm, and a diameter (C) of 48 mm was placed. These dimensions of the acoustic transmission medium 13 are the dimensions of the portions illustrated in Fig. 5.

The cell detachment apparatus was assembled as illustrated in Figs. 1A and 1B, and the 60-mm diameter dish manufactured by Corning Incorporated as the culture container 21 in which cells were cultured was placed on the silicone rubber placed on the vibrating plate 11. At this time, the dish was placed while aligning the center of the dish with the center of the acoustic transmission medium 13 using the positioning pin 24. At this time, the close contact area of the acoustic transmission medium and the culture container was 30%. The close contact area refers to the proportion of the area of the region where the acoustic transmission medium 13 and the culture container 21 are in contact with each other to the area of the region inside the protruding portion 23 of the bottom surface of the culture container 21.

In the culture of cells, Chinese hamster ovary (CHO) cells were seeded at a density of 10000 cells/cm$^2$ in the 60-mm diameter dish manufactured by Corning Incorporated and cultured under an environment with a temperature of 37°C and a $CO_2$ concentration of 5%. A culture medium obtained by adding 10% of fetal bovine serum (manufactured by Sigma-Aldrich Co. LLC) and 1% of penicillin-streptomycin (10000 U/ml manufactured by Thermo Fisher Scientific Inc.) to Ham's F12 (manufactured by Thermo Fisher Scientific Inc.) was used. The culture was performed for 48 hours, the state of cells was observed using a phase contrast microscope, and the adhesion and the proliferation of cells were confirmed. The cell occupancy area ratio of the dish was about eight tenths.

(Detaching of Cells)

The culture medium in the dish was removed, and the dish was washed with phosphate-buffered saline (PBS(-) manufactured by Thermo Fisher Scientific Inc.). Then, phosphate-buffered saline (PBS(-) manufactured by Thermo Fisher Scientific Inc.) was added as a detachment solution into the dish, and the dish was immersed for 3 minutes.

Then, the culture container was placed in the cell detachment apparatus, and cells were detached by frequency sweep vibration (frequencies of 22 to 27 kHz, a frequency sweep period of 1 s, and a voltage of 100 V) for 1 minute and 30 seconds in the standing wave mode at an environmental temperature of 37°C. Then, phases were switched, and cells were detached by frequency sweep vibration (frequencies of 22 to 27 kHz, a frequency sweep period of 1 s, and a voltage of 100 V) for 1 minute and 30 seconds in the traveling wave mode.

After the detached cells were collected, the number of cells was measured using a hemocytometer, and the survival rate was calculated using a vitality assay based on the trypan blue staining. First, a suspension of the detached cells and 0.4% trypan blue solution were mixed at 1:1, and dead cells included in the suspension were stained. Next, the suspension was injected into a hemocytometer (DHC-N01 manufactured by NanoEntek), and the numbers of live cells and dead cells on the grid of the hemocytometer were counted by microscope observation. Among the cells detached by the vibration of the cell detachment apparatus, the proportion of the number of counted live cells to the number of all the cells was calculated and determined as the survival rate.

From the dish after the ultrasonic detaching, cells that are not detached by the ultrasound using a cell scraper were also all detached. A suspension of the detached cell was injected into the hemocytometer (DHC-N01 manufactured by NanoEntek) and observed using a microscope, whereby the number of detached cells was measured. The number of all the cells detached by the ultrasound and the number of cells detached by the cell scraper after that were combined into the total number of detached cells, and the proportion of the number of cells detached by the ultrasound to the total number of detached cells was defined as the cell detachment efficiency and calculated. As a result, the survival rate was 98.5%, and the cell detachment efficiency was 94.9%. If both the survival rate and the cell detachment efficiency were greater than or equal to 90%, it was determined that the result was excellent.

(Second Exemplary Embodiment)

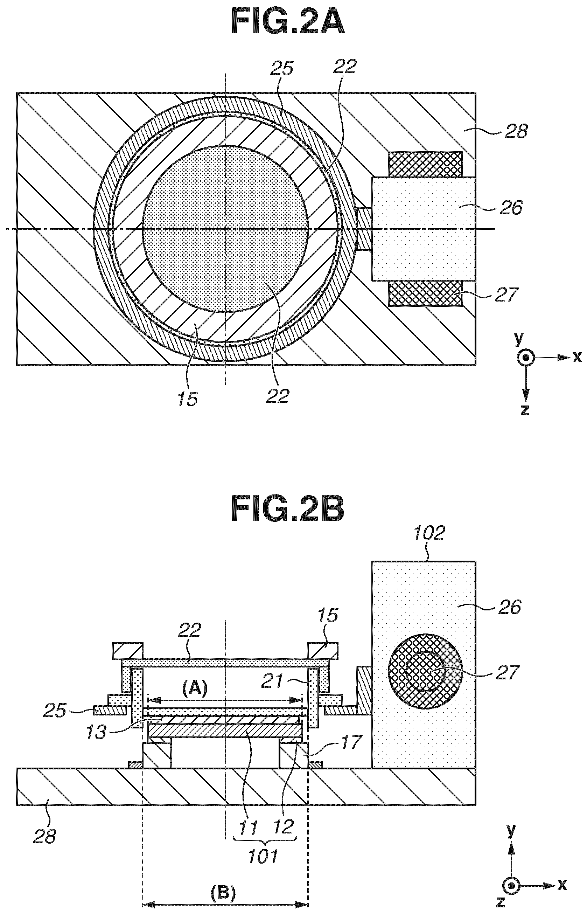

Schematic diagrams in Figs. 2A and 2B illustrate a cell detachment apparatus including a vibrating plate 11 having external dimensions with which the vibrating plate 11 does not come into contact with a protruding portion 23 of a culture container 21. In a second exemplary embodiment, it is desirable that the diameter of the vibrating plate 11 indicated by (A) in Fig. 2B be smaller than the inner diameter of the culture container 21 indicated by (B) in Fig. 2B. A cross section of the vibrating plate 11 in a plane perpendicular to the direction in which a vibrator, the vibrating plate, an acoustic transmission medium, and the culture container are arranged to be stacked has a circular shape, and the diameter of the vibrating plate 11 refers to the diameter of the cross section.

That the protruding portion 23 of the culture container 21 and the vibrating plate 11 do not come into contact with each other means that the protruding portion 23 of the culture container 21 does not substantially come into direct contact with a vibrating surface of the vibrating plate 11. It is desirable that the protruding portion 23 be in midair. A buffer material capable of preventing the vibration of the vibrating plate 11 from being transmitted to the culture container 21 through the protruding portion 23 may be present. That is, the cell detachment apparatus may be configured to bring at least one of the acoustic transmission medium and the vibrating plate into contact with the protruding portion through the buffer material, and prevent the acoustic transmission medium and the vibrating plate from coming into contact with the protruding portion not through the buffer material. It is desirable that the buffer material be at least one of felt and sponge.

Further, in other words, it is desirable that the specific gravity of a substance present between the protruding portion and the vibrating plate at the position where the protruding portion and the vibrating plate are closest to each other be greater than 0 and smaller than 0.90. The specific gravity of the substance present between the protruding portion and the vibrating plate is smaller than 0.90, whereby the vibration is less likely to be transmitted. Thus, it is possible to reduce the interference of the vibration. It is desirable that the specific gravity of the substance present between the protruding portion and the vibrating plate be smaller than 0.80. It is desirable that the specific gravity of the substance present between the protruding portion and the vibrating plate be smaller than 0.50.

For example, in a 60-mm diameter dish manufactured by Thermo Fisher Scientific Inc. in table 1, the inner diameter of the culture container is 52.2 mm. Thus, if the outer diameter of the vibrating plate is less than or equal to a diameter of 50 mm, the vibrating plate and the protruding portion 23 do not substantially come into contact with each other. That is, the outer diameter of the vibrating plate is smaller than the diameter of a region inside the protruding portion 23 located in an outer peripheral portion of the bottom surface of the dish in the bottom surface of the dish, whereby it is possible to prevent the vibrating plate 11 and the protruding portion 23 from coming into direct contact with each other.

Although a case has been described where the protruding portion 23 is provided in an approximately circular shape on the bottom surface of the dish and the vibrating plate 11 has a disk-like shape, the shapes of the protruding portion 23 and the vibrating plate 11 are not limited to these, and the vibrating plate 11 and the protruding portion 23 only need to avoid overlapping each other. That is, the position where the position of the protruding portion is projected onto the surface on which the vibrating plate is present in the direction in which the vibrator, the vibrating plate, the acoustic transmission medium, and the culture container are arranged to be stacked (a y-direction in Figs. 2A and 2B) and the vibrating plate only need to avoid overlapping each other. With such a structure, it is possible to directly lift a lower portion of the culture container after a cell detachment process and easily detach the culture container from the cell detachment apparatus.

(Example 2)

In example 2, as the culture container 21, a 60-mm diameter dish Nunc manufactured by Thermo Fisher Scientific Inc. in table 1 was used. As the vibrating plate 11 of the cell detachment apparatus, a glass plate was used which had an outer diameter of 50 mm and a thickness of 2 mm and did not come into contact with the protruding portion 23. The main component of the piezoelectric material included in the vibrator 12 was $BaTiO_3$. The vibrator 12 had an outer diameter of 50 mm, which was the same as that of the vibrating plate, and an inner diameter of 37 mm. The vibrator 12 had a thickness of 1 mm, considering that the neutral surface was on the vibrating plate 11 side. On the vibrating plate 11, as the acoustic transmission medium 13, silicone rubber with a hardness of 10° having a central convex disk shape and having a center height (A) of 0.8 mm, a side surface height (B) of 0.4 mm, and a diameter (C) of 48 mm was placed. These dimensions of the acoustic transmission medium 13 are the dimensions of the portions illustrated in Fig. 5. The cell detachment apparatus was assembled as illustrated in Figs. 2A and 2B, and the 60-mm diameter dish Nunc as the culture container 21 in which cells were cultured was installed on the silicone rubber installed on the vibrating plate 11. At this time, the culture container 21 was installed by aligning the center of the acoustic transmission medium 13 and the center of the culture container 21 using a positioning jig 102. At this time, the close contact area of the acoustic transmission medium 13 and the culture container 21 was 85%. The close contact area refers to the proportion of the area of the region where the acoustic transmission medium 13 and the culture container 21 are in contact with each other to the area of the region inside the protruding portion 23 of the bottom surface of the culture container 21.

(Detaching of Cells)

A culture medium in the dish was removed, and the dish was washed with phosphate-buffered saline (PBS(-) manufactured by Thermo Fisher Scientific Inc.). Then, phosphate-buffered saline (PBS(-) manufactured by Thermo Fisher Scientific Inc.) was added as a detachment solution into the dish, and the dish was immersed for 3 minutes.

Then, the culture container was installed in the cell detachment apparatus, and cells were detached by frequency sweep vibration (frequencies of 22 to 27 kHz, a frequency sweep period of 1 s, and a voltage of 100 V) for 3 minutes in the standing wave mode at an environmental temperature of 37°C.

The detached cells were inspected by a method similar to that in example 1. As a result, the survival rate was 98.0%, and the cell detachment efficiency was 95.7%. If both the survival rate and the cell detachment efficiency were greater than or equal to 90%, it was determined that the result was excellent.

(Third Exemplary Embodiment)

Schematic diagrams in Figs. 3A and 3B are schematic diagrams of a cell detachment apparatus configured so that in a case where a culture container 21 is placed on an acoustic transmission medium 13, a vibrating plate 11 and a protruding portion 23 do not come into contact with each other because the thickness of the acoustic transmission medium 13 is greater than the length of the protruding portion 23.

That the protruding portion 23 of the culture container 21 and the vibrating plate 11 do not come into contact with each other means that the protruding portion 23 of the culture container 21 does not substantially come into direct contact with a vibrating surface of the vibrating plate 11. It is desirable that the protruding portion 23 be in midair. A buffer material capable of preventing the vibration of the vibrating plate 11 from being transmitted to the culture container 21 through the protruding portion 23 may be present. That is, the cell detachment apparatus may be configured to bring at least one of the acoustic transmission medium and the vibrating plate into contact with the protruding portion through the buffer material, and prevent the acoustic transmission medium and the vibrating plate from coming into contact with the protruding portion not through the buffer material. It is desirable that the buffer material be at least one of felt and sponge.

Further, in other words, it is desirable that the specific gravity of a substance present between the protruding portion and the vibrating plate at the position where the protruding portion and the vibrating plate are closest to each other be greater than 0 and smaller than 0.90. The specific gravity of the substance present between the protruding portion and the vibrating plate is smaller than 0.90, whereby the vibration is less likely to be transmitted. Thus, it is possible to reduce the interference of the vibration. It is desirable that the specific gravity of the substance present between the protruding portion and the vibrating plate be smaller than 0.80. It is desirable that the specific gravity of the substance present between the protruding portion and the vibrating plate be smaller than 0.50.

In a third exemplary embodiment, it is desirable that the thickness of the acoustic transmission medium 13 indicated by (A) in Fig. 3B in a case where the culture container 21 is placed on the acoustic transmission medium 13 be greater than the length of the protruding portion 23 of the culture container 21 indicated by (B) in Fig. 3B. If the culture container 21 is placed on the acoustic transmission medium 13, the acoustic transmission medium 13 is compressed. If, however, the thickness of the acoustic transmission medium 13 at this time is greater than the length of the protruding portion 23 of the culture container 21, the protruding portion 23 does not come into direct contact with the vibrating plate 11. The thickness of the acoustic transmission medium refers to the thickness of the acoustic transmission medium in the direction in which a vibrator, the vibrating plate, the acoustic transmission medium, and the culture container are arranged to be stacked. If the volume of the acoustic transmission medium 13 itself is great, the transmission efficiency of the vibration may be low. Thus, it is desirable that the hardness of the acoustic transmission medium 13 to be used be high.

To obtain a structure where the protruding portion 23 of the culture container 21 does not come into contact with the vibrating plate 11, the thickness of the acoustic transmission medium 13 is determined considering that the length of the protruding portion 23 of the culture container 21 of each company is 0.5 mm, and also considering a load using a weight 15 in a cell detachment step. It is desirable that the thickness of the acoustic transmission medium 13 provided on the vibrating plate 11 be greater than or equal to 0.8 mm. If ultrasound transmission properties are considered, a vibration greatly attenuates in a polymeric material, and therefore, it is desirable that the thickness of the acoustic transmission medium 13 be less than or equal to 1.5 mm.

(Example 3)

In example 3, as the culture container 21, a 60-mm diameter dish Iwaki manufactured by AGC Techno Glass Co., Ltd. in table 1 was used. As the vibrating plate 11 of the cell detachment apparatus, a vibrating plate was used which had an outer diameter of 70 mm and a thickness of 4 mm. The material of the vibrating plate was quartz. The material composition of the piezoelectric material included in the vibrator 12 was PZT. The vibrator 12 had an outer diameter of 70 mm, which was the same as that of the vibrating plate 11, and an inner diameter of 57 mm. The vibrator 12 had a thickness of 2 mm, considering that the neutral surface was on the vibrating plate 11 side. On the vibrating plate 11, as the acoustic transmission medium 13, urethane with a hardness of 50° having a central convex disk shape and having a center height (A) of 0.7 mm, a side surface height (B) of 0.3 mm, and a diameter (C) of 48 mm was installed. These dimensions of the acoustic transmission medium 13 are the dimensions of the portions illustrated in Fig. 5. The cell detachment apparatus was assembled as illustrated in Figs. 1A and 1B, and the 60-mm diameter dish Iwaki as the culture container 21 in which cells were cultured was installed on the urethane installed on the vibrating plate 11. At this time, the culture container 21 was installed while aligning the center of the culture container 21 with the center of the acoustic transmission medium 13 using a positioning pin 24. The close contact area of the acoustic transmission medium 13 and the culture container 21 was 97%. The close contact area refers to the proportion of the area of the region where the acoustic transmission medium 13 and the culture container 21 are in contact with each other to the area of the region inside the protruding portion 23 of the bottom surface of the culture container 21.

The culture of CHO cells, a cell detachment step, and the inspection of detached cells were performed by methods similar to those in example 2.

As a result, the survival rate was 99.0%, and the cell detachment efficiency was 93.0%. If both the survival rate and the cell detachment efficiency were greater than or equal to 90%, it was determined that the result was excellent.

Exemplary embodiments of the present invention may also be implemented by a computer of a system or apparatus (e.g., an application specific integrated circuit (ASIC)) that reads and executes computer-executable instructions (e.g., one or more programs) recorded on a storage medium performing one or more of the exemplary embodiments, and / or by a computer of a system or apparatus including one or more circuits that perform one or more functions of the exemplary embodiment, and for example, by reading and executing computer-executable instructions from a storage medium to perform the one or more functions of the exemplary embodiments, and / or by a method performed by a computer of a system or apparatus by controlling one or more circuits to perform the one or more functions of the exemplary embodiments. The computer may include one or more processors (e.g., central processing unit (CPU), micro processing unit (MPU)) and may include a network of a separate computer or a separate processor to read and execute the computer executable instructions. The computer executable instructions may be provided to the computer, for example, from a network or a storage medium. The storage medium may include one or more of a hard disk, a random access memory (RAM), a read-only memory (ROM), a storage device of a distributed computing system, an optical disc (a compact disc (CD), a digital versatile disc (DVD), a Blu-ray disc (BD), etc.), a flash memory device, a memory card, and the like.

The disclosure of the present exemplary embodiments includes the following Configurations and Methods.

[Configuration 1]

A cell detachment apparatus that detaches cells placed on a culture surface of a culture container for cells by applying a vibration to the cells, the cell detachment apparatus comprising:

a vibrator; and
a vibrating plate,
wherein the culture container includes a protruding portion on a surface on an opposite side of the culture surface, and
wherein the cell detachment apparatus is configured so that in a case where the vibrator, the vibrating plate, an acoustic transmission medium, and the culture container are arranged to be stacked in this order, the acoustic transmission medium and the vibrating plate do not come into contact with the protruding portion.

[Configuration 2]

The cell detachment apparatus according to Configuration 1, wherein the cell detachment apparatus is configured so that in the case where the vibrator, the vibrating plate, the acoustic transmission medium, and the culture container are arranged to be stacked in this order, at least one of the acoustic transmission medium and the vibrating plate comes into contact with the protruding portion through a buffer material.

[Configuration 3]

The cell detachment apparatus according to Configuration 2, wherein the buffer material includes at least one of felt and sponge.

[Configuration 4]

The cell detachment apparatus according to any one of Configurations 1 to 3, wherein the vibration is a vibration in an ultrasonic range.

[Configuration 5]

The cell detachment apparatus according to any one of Configurations 1 to 4, wherein in the case where the vibrator, the vibrating plate, the acoustic transmission medium, and the culture container are arranged to be stacked in this order, the vibrating plate includes a groove in a region including a position where a position of the protruding portion is projected onto the vibrating plate in a direction in which the vibrator, the vibrating plate, the acoustic transmission medium, and the culture container are arranged to be stacked.

[Configuration 6]

The cell detachment apparatus according to Configuration 5, wherein a depth of the groove in the direction in which the vibrator, the vibrating plate, the acoustic transmission medium, and the culture container are arranged to be stacked is greater than a length of the protruding portion in the direction in which the vibrator, the vibrating plate, the acoustic transmission medium, and the culture container are arranged to be stacked.

EP 4 613 843 A1

[Configuration 7]

The cell detachment apparatus according to Configuration 5 or 6, wherein a width of the groove in the direction in which the vibrator, the vibrating plate, the acoustic transmission medium, and the culture container are arranged to be stacked is wider than a width of the protruding portion in a direction perpendicular to the direction in which the vibrator, the vibrating plate, the acoustic transmission medium, and the culture container are arranged to be stacked.

[Configuration 8]

The cell detachment apparatus according to any one of Configurations 1 to 4, wherein in a case where the vibrator, the vibrating plate, the acoustic transmission medium, and the culture container are arranged to be stacked in this order, a position where a position of the protruding portion is projected onto a surface on which the vibrating plate is present and the vibrating plate do not overlap with each other in a direction in which the vibrator, the vibrating plate, the acoustic transmission medium, and the culture container are arranged to be stacked.

[Configuration 9]

The cell detachment apparatus according to Configuration 8,

wherein in a plane perpendicular to the direction in which the vibrator, the vibrating plate, the acoustic transmission medium, and the culture container are arranged to be stacked, a cross section of the vibrating plate has a circular shape, and
wherein an outer diameter of the cross section of the vibrating plate is smaller than a diameter of a region inside the protruding portion in the surface on the opposite side of the culture surface of the culture container.

[Configuration 10]

The cell detachment apparatus according to any one of Configurations 1 to 9, wherein the vibrating plate includes at least one selected from the group consisting of glass, metal, and quartz.

[Configuration 11]

The cell detachment apparatus according to any one of Configurations 1 to 10, wherein in a case where the culture container is placed on the acoustic transmission medium, a thickness of the acoustic transmission medium in a direction in which the vibrator, the vibrating plate, the acoustic transmission medium, and the culture container are arranged to be stacked is greater than a height of the protruding portion in the direction in which the vibrator, the vibrating plate, the acoustic transmission medium, and the culture container are arranged to be stacked.

[Configuration 12]

The cell detachment apparatus according to any one of Configurations 1 to 11, wherein the cell detachment apparatus is configured so that in a case where the vibrator, the vibrating plate, the acoustic transmission medium, and the culture container are arranged to be stacked in this order, a proportion of an area of a region where the culture container is in contact with the acoustic transmission medium to an area of a region inside the protruding portion on the surface on the opposite side of the culture surface of the culture container is greater than or equal to 30% and less than 100%.

[Configuration 13]

The cell detachment apparatus according to any one of Configurations 1 to 12, wherein a surface of the acoustic transmission medium that comes into contact with the culture container includes a convex curved surface.

[Configuration 14]

The cell detachment apparatus according to any one of Configurations 1 to 13, wherein an outer surface of a surface of the acoustic transmission medium that comes into contact with the culture container has recesses and projections.

[Configuration 15]

The cell detachment apparatus according to any one of Configurations 1 to 14, wherein the acoustic transmission medium includes a polymeric material.

[Configuration 16]

The cell detachment apparatus according to Configuration 15, wherein the polymeric material includes at least one selected from the group consisting of silicone resin, urethane resin, diallyphthalate resin, unsaturated polyester, epoxy resin, phenolic resin, urea resin, melamine resin, and natural rubber.

[Configuration 17]

The cell detachment apparatus according to Configuration 15 or 16, wherein the polymeric material includes silicone rubber.

[Configuration 18]

A cell detachment apparatus that detaches cells placed on a culture surface of a culture container for cells by applying a vibration to the cells, the cell detachment apparatus comprising:

a vibrator; and
a vibrating plate,
wherein in a case where the vibrator, the vibrating plate, an acoustic transmission medium, and the culture container are arranged to be stacked in this order, the culture container includes a protruding portion on a surface on an opposite side of the culture surface, and
wherein a specific gravity of a substance present between the protruding portion and the vibrating plate at a position where the protruding portion and the vibrating plate are closest to each other is greater than 0 and smaller than 0.90.

[Method 1]

A method for detaching cells placed on a culture surface of a culture container for cells by applying a vibration to the cells, the method comprising:

through use of a cell detachment apparatus including the culture container including a protruding portion on a surface on an opposite side of the culture surface, an acoustic transmission medium, a vibrator, and a vibrating plate, applying a vibration to the cells in a state where the vibrator, the vibrating plate, the acoustic transmission medium, and the culture container are arranged to be stacked in this order; and
applying a vibration to the cells in a state where the acoustic transmission medium and the vibrating plate are not in contact with the protruding portion.

[Method 2]

A method for detaching cells placed on a culture surface of a culture container for cells by applying a vibration to the cells, the method comprising:

through use of a cell detachment apparatus including the culture container including a protruding portion on a surface on an opposite side of the culture surface of the culture container, an acoustic transmission medium, a vibrator, and a vibrating plate, applying a vibration to the cells in a state where the vibrator, the vibrating plate, the acoustic transmission medium, and the culture container are arranged to be stacked in this order; and
applying a vibration to the cells in a state where a specific gravity of a substance present between the protruding portion and the vibrating plate at a position where the protruding portion and the vibrating plate are closest to each other is greater than 0 and smaller than 0.90.

The present invention is not limited to the above exemplary embodiments, and various modifications and variations can be made without departing from the spirit and scope of the present invention.
This application claims the benefit of Japanese Patent Application No. 2022-175807, filed November 1, 2022, which is hereby incorporated by reference herein in its entirety.

Reference Signs List

11 Vibrating plate
12 Vibrator
13 Acoustic transmission medium
14 Groove
15 Weight
16 Buffer material
17 Lower cover
18 Upper cover
19 Bolt
21 Culture container
22 Culture container upper lid
23 Protruding portion
24 Positioning pin
25 Annular holding ring
26 Z-axis stage bracket
27 Dial
28 Bottom plate
29 Pedestal positioner
101 Vibration generation unit
102 Positioning jig

**Claims**

1. A cell detachment apparatus that detaches cells placed on a culture surface of a culture container for cells by applying a vibration to the cells, the cell detachment apparatus comprising:

   a vibrator; and
   a vibrating plate,
   wherein the culture container includes a protruding portion on a surface on an opposite side of the culture surface, and
   wherein the cell detachment apparatus is configured so that in a case where the vibrator, the vibrating plate, an acoustic transmission medium, and the culture container are arranged to be stacked in this order, the acoustic transmission medium and the vibrating plate do not come into contact with the protruding portion.

2. The cell detachment apparatus according to Claim 1, wherein the cell detachment apparatus is configured so that in the case where the vibrator, the vibrating plate, the acoustic transmission medium, and the culture container are arranged to be stacked in this order, at least one of the acoustic transmission medium and the vibrating plate comes into contact with the protruding portion through a buffer material.

3. The cell detachment apparatus according to Claim 2, wherein the buffer material includes at least one of felt and sponge.

4. The cell detachment apparatus according to Claim 1 or 2, wherein the vibration is a vibration in an ultrasonic range.

5. The cell detachment apparatus according to Claim 1 or 2, wherein in the case where the vibrator, the vibrating plate, the acoustic transmission medium, and the culture container are arranged to be stacked in this order, the vibrating plate includes a groove in a region including a position where a position of the protruding portion is projected onto the vibrating plate in a direction in which the vibrator, the vibrating plate, the acoustic transmission medium, and the culture container are arranged to be stacked.

6. The cell detachment apparatus according to Claim 5, wherein a depth of the groove in the direction in which the vibrator, the vibrating plate, the acoustic transmission medium, and the culture container are arranged to be stacked is greater than a length of the protruding portion in the direction in which the vibrator, the vibrating plate, the acoustic transmission medium, and the culture container are arranged to be stacked.

7.  The cell detachment apparatus according to Claim 5, wherein a width of the groove in the direction in which the vibrator, the vibrating plate, the acoustic transmission medium, and the culture container are arranged to be stacked is wider than a width of the protruding portion in a direction perpendicular to the direction in which the vibrator, the vibrating plate, the acoustic transmission medium, and the culture container are arranged to be stacked.

8.  The cell detachment apparatus according to Claim 1 or 2, wherein in a case where the vibrator, the vibrating plate, the acoustic transmission medium, and the culture container are arranged to be stacked in this order, a position where a position of the protruding portion is projected onto a surface on which the vibrating plate is present and the vibrating plate do not overlap with each other in a direction in which the vibrator, the vibrating plate, the acoustic transmission medium, and the culture container are arranged to be stacked.

9.  The cell detachment apparatus according to Claim 8,

    wherein in a plane perpendicular to the direction in which the vibrator, the vibrating plate, the acoustic transmission medium, and the culture container are arranged to be stacked, a cross section of the vibrating plate has a circular shape, and
    wherein an outer diameter of the cross section of the vibrating plate is smaller than a diameter of a region inside the protruding portion in the surface on the opposite side of the culture surface of the culture container.

10. The cell detachment apparatus according to Claim 1 or 2, wherein the vibrating plate includes at least one selected from the group consisting of glass, metal, and quartz.

11. The cell detachment apparatus according to Claim 1 or 2, wherein in a case where the culture container is placed on the acoustic transmission medium, a thickness of the acoustic transmission medium in a direction in which the vibrator, the vibrating plate, the acoustic transmission medium, and the culture container are arranged to be stacked is greater than a height of the protruding portion in the direction in which the vibrator, the vibrating plate, the acoustic transmission medium, and the culture container are arranged to be stacked.

12. The cell detachment apparatus according to Claim 1 or 2, wherein the cell detachment apparatus is configured so that in a case where the vibrator, the vibrating plate, the acoustic transmission medium, and the culture container are arranged to be stacked in this order, a proportion of an area of a region where the culture container is in contact with the acoustic transmission medium to an area of a region inside the protruding portion on the surface on the opposite side of the culture surface of the culture container is greater than or equal to 30% and less than 100%.

13. The cell detachment apparatus according to Claim 1 or 2, wherein a surface of the acoustic transmission medium that comes into contact with the culture container includes a convex curved surface.

14. The cell detachment apparatus according to Claim 1 or 2, wherein an outer surface of a surface of the acoustic transmission medium that comes into contact with the culture container has recesses and projections.

15. The cell detachment apparatus according to Claim 1 or 2, wherein the acoustic transmission medium includes a polymeric material.

16. The cell detachment apparatus according to Claim 15, wherein the polymeric material includes at least one selected from the group consisting of silicone resin, urethane resin, diallyphthalate resin, unsaturated polyester, epoxy resin, phenolic resin, urea resin, melamine resin, and natural rubber.

17. The cell detachment apparatus according to Claim 16, wherein the polymeric material includes silicone rubber.

18. A cell detachment apparatus that detaches cells placed on a culture surface of a culture container for cells by applying a vibration to the cells, the cell detachment apparatus comprising:

    a vibrator; and
    a vibrating plate,
    wherein in a case where the vibrator, the vibrating plate, an acoustic transmission medium, and the culture container are arranged to be stacked in this order, the culture container includes a protruding portion on a surface on an opposite side of the culture surface, and

wherein a specific gravity of a substance present between the protruding portion and the vibrating plate at a position where the protruding portion and the vibrating plate are closest to each other is greater than 0 and smaller than 0.90.

19. A method for detaching cells placed on a culture surface of a culture container for cells by applying a vibration to the cells, the method comprising:

through use of a cell detachment apparatus including the culture container including a protruding portion on a surface on an opposite side of the culture surface, an acoustic transmission medium, a vibrator, and a vibrating plate, applying a vibration to the cells in a state where the vibrator, the vibrating plate, the acoustic transmission medium, and the culture container are placed in this order; and

applying a vibration to the cells in a state where the acoustic transmission medium and the vibrating plate are not in contact with the protruding portion.

20. A method for detaching cells placed on a culture surface of a culture container for cells by applying a vibration to the cells, the method comprising:

through use of a cell detachment apparatus including the culture container including a protruding portion on a surface on an opposite side of the culture surface of the culture container, an acoustic transmission medium, a vibrator, and a vibrating plate, applying a vibration to the cells in a state where the vibrator, the vibrating plate, the acoustic transmission medium, and the culture container are arranged to be stacked in this order; and

applying a vibration to the cells in a state where a specific gravity of a substance present between the protruding portion and the vibrating plate at a position where the protruding portion and the vibrating plate are closest to each other is greater than 0 and smaller than 0.90.

# FIG.1A

# FIG.1B

# FIG.2A

# FIG.2B

# FIG.3A

# FIG.3B

# FIG.4

# FIG.5

# FIG.6

# FIG.7A

# FIG.7B

## INTERNATIONAL SEARCH REPORT

<table>
<tr><td>International application No.</td></tr>
<tr><td><strong>PCT/JP2023/038841</strong></td></tr>
</table>

| A. | CLASSIFICATION OF SUBJECT MATTER |
|---|---|

*C12N 1/02*(2006.01)i; *C12M 1/00*(2006.01)i
FI: C12M1/00 D; C12N1/02

According to International Patent Classification (IPC) or to both national classification and IPC

| B. | FIELDS SEARCHED |
|---|---|

Minimum documentation searched (classification system followed by classification symbols)

C12N1/02; C12M1/00

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

| C. | DOCUMENTS CONSIDERED TO BE RELEVANT |
|---|---|

| Category* | Citation of document, with indication, where appropriate, of the relevant passages | Relevant to claim No. |
|---|---|---|
| A | JP 2015-29508 A (ASAHI KASEI CORP.) 16 February 2015 (2015-02-16) | 1-20 |
| A | JP 2005-80606 A (BIO MAP CO.) 31 March 2005 (2005-03-31) | 1-20 |
| A | JP 2019-97478 A (TOYOBO CO., LTD.) 24 June 2019 (2019-06-24) | 1-20 |
| A | WO 2017/154197 A1 (HITACHI, LTD.) 14 September 2017 (2017-09-14) | 1-20 |

☐ Further documents are listed in the continuation of Box C.  ☑ See patent family annex.

| * | Special categories of cited documents: |
|---|---|
| "A" | document defining the general state of the art which is not considered to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
|---|---|
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| **06 December 2023** | **19 December 2023** |

| Name and mailing address of the ISA/JP | Authorized officer |
|---|---|
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (January 2015)

## INTERNATIONAL SEARCH REPORT
### Information on patent family members

International application No.

**PCT/JP2023/038841**

| Patent document cited in search report | | | Publication date (day/month/year) | Patent family member(s) | | | Publication date (day/month/year) |
|---|---|---|---|---|---|---|---|
| JP | 2015-29508 | A | 16 February 2015 | (Family: none) | | | |
| JP | 2005-80606 | A | 31 March 2005 | (Family: none) | | | |
| JP | 2019-97478 | A | 24 June 2019 | (Family: none) | | | |
| WO | 2017/154197 | A1 | 14 September 2017 | JP | 17-154197 | A1 | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2008079554 A

- JP 2022175807 A